# EUROPÄISCHE PATENTANMELDUNG

(11) **EP 1 181 889 A1**
(43) Veröffentlichungstag der Anmeldung: **27.02.2002**
(21) Anmeldenummer: 01119591.4
(22) Anmeldetag: 16.08.2001
(51) Int. Cl.: A61B 5/03, A61M 3/02, A61M 1/00

(54) **Katheter und Verfahren zu seiner Anwendung**

(30) Priorität: 17.08.2000 DE 10040164
(71) Anmelder: REHAU AG + Co, 95111 Rehau (DE)
(72) Erfinder: Bayyoud, Yousef, Dr., 95111 Rehau (DE); Gropp, Friedrich, Dr., 95111 Rehau (DE)

(57) **Zusammenfassung**

Die Erfindung betriift einen Katheter für die Behandlung von Flüssigkeitsansammlungen in Hohlräumen zwischen den Hirnhäuten, in Ventrikein und/oder Subarachnoidalräumen sowie dem Hirngewebe mit Fluiden, bestehend aus polymeren Werkstoff, wobei der Katheter wenigsten 2 Lumen aufweist, wobei in jedes Lumen am freien Ende des Katheters wenigstens eine Öffnung eingebracht ist, wobei am freien Ende des Katheters ein Druckmesselement positioniert ist, welches von den Öffnungen der Lumen beabstandet ist und wobei die Lumen so dimensioniert sind, dass die Druckverhältnisse in den Hohlräumen zwischen den Hirnhäuten, in den Ventrikeln und/oder Subarachnoidalräumen bei Zuführung und oder Abführung von Fluiden annährend konstant sind.

## Beschreibung

Katheter für die Behandlung von Flüssigkeitsansammlungen in Hohlräumen zwischen den Hirnhäuten, in Ventrikeln und/oder Subarachnoidalräumen sowie dem Hirngewebe mit Fluiden.

In der neurochirurgischen, neurologischen, neonatologischen und pädiatrischen Therapie ist der Einsatz von Kathetern für die Behandlung von Flüssigkeitsansammlungen in Hohlräumen zwischen den Hirnhäuten, in Ventrikeln und/oder Subarachnoidalräumen bzw. des Hirngewebes bei einer Vielzahl von Erkrankungen mit Hirndrucksymptomatik durch eine externe Liquordrainage bekannt.
Diese bekannten Einmalsysteme sind nicht für eine ständige Überwachung des Liquordruckes über einen Monitor bei gleichzeitiger Drainage ausgelegt.

Für eine ständige Drucküberwachung und Drainage sind Mikrochipkatheter oder fieberoptische Katheter bekannt, deren Anschaffung jedoch sehr kostenintensiv ist und die mit einer aufwendigen Messtechnik versehen demzufolge nur bei besonderen Erkrankungen eingesetzt werden.
Die erforderliche und notwendige Messung des intracraniellen Druckes erfolgt mittels eines externen Druckwandlersystemes.

Bei diesen Kathetern aus dem bekannten Stand der Technik ist es nachteilig, dass eine gleichzeitige Druckmessung und Drainage nicht möglich ist.
Ein weiterer Nachteil besteht darin, dass bei der Anwendung im Bereich des menschlichen Gehirns der Katheter mittels geeigneter technischer Maßnahmen lokalisierbar sein muss. Dies ist bisher nur mit solchen Kathetern möglich, die durch den Einsatz bestimmter chemischer Stoffe kontrastiert sind.

Diese Kontrastierung führt jedoch zu einer rauen Oberflächenstruktur der Katheter mit den Nachteilen der Anlagerung von Proteinen, Bakterien und Zellbestandteilen.
Weiterhin ist eine optische Kontrolle der Fluiden durch diese Kontrastierung der Katheter nicht möglich.

Die Katheter werden derzeit so verwendet, dass diese mittels spezieller und aufwendiger Verbindungselemente den erforderlichen Funktionen Druckmessung und Drainage nacheinander zugeführt werden.

Die damit verbundenen Probleme und Schwierigkeiten, insbesondere die Diskonnektionsmöglichkeit mit der Gefahr der Bakterieninfektion des Gehirnes stellt einen schwerwiegenden und nicht akzeptierbaren Nachteil dar.
Außerdem wird die medizinisch geforderte kontinuierliche Aufzeichnung des intracraniellen Druckes durch die Drainagezyklen unterbrochen.

Die Erfindung hat sich nun die Aufgabe gestellt, die Nachteile des bekannten Standes der Technik zu vermeiden und einen Katheter aufzuzeigen, der einfach handhabbar und wirtschaftlich herstellbar ist und bei dem gleichzeitig mit der Zuführung und dem Abfluss von Fluiden eine synchrone Überwachung des intracraniellen Druckes kontinuierlich und jederzeit möglich ist.
Eine weitere Aufgabe besteht darin, eine optische Kontrolle der zuzuführenden bzw. abzufließenden Fluide zu erreichen und die Lage des Katheters jederzeit mit der geeigneten Technik lokalisieren zu können, ohne dass es zu Artefakten oder Auslöschen von Hirnstrukturen aufgrund von Überlagerungen durch das Kathetermaterial kommt.

Erfindungsgemäß wird dies dadurch gelöst, dass der Katheter aus einem polymeren Werkstoff besteht, wobei dieser wenigstens zwei Lumen aufweist, wobei in jedes Lumen am freien Ende des Katheters wenigstens eine Öffnung eingebracht ist, am freien Ende des Katheters ein Druckmesselement positioniert ist, welches von den Öffnungen der Lumen beabstandet ist und wobei die Lumen so dimensioniert sind, dass die Druckverhältnisse in den Hohlräumen zwischen den Hirnhäuten in Ventrikeln und/oder Subarachnoidalräumen sowie dem Hirngewebe bei der Zuführung und Abführung von Fluiden annähernd konstant sind.

Weitere vorteilhafte Ausgestaltungen sind den Unteransprüchen zu entnehmen.

Vorteilhaft wird hier gesehen, dass mit dem erfindungsgemäßen Katheter eine kontinuierliche bzw. alternierende Befüllung und Drainage von Lösungen und Medikamenten in den Hohlräumen zwischen den Hirnhäuten, in Ventrikeln und/oder Subarachnoidalräumen bzw. des Hirngewebes unter einer permanenten Messung des intracraniellen Druckes erfolgen kann.
Die Messung des intracraniellen Druckes in der Gewebeflüssigkeit garantiert die zuverlässige Abstimmung von Füllgeschwindigkeit und Drainagegeschwindigkeit und erkennt sofort gefährliche intracranielle Druckveränderungen, welche durch Veränderungen der Füllgeschwindigkeit bzw. der Drainagegeschwindigkeit entgegengewirkt werden kann.

Mit dem erfindungsgemäßen Katheter ist somit ein druckkontrolliertes kontinuierliches Füllen in den Hohlräumen zwischen den Hirnhäuten, von Ventrikeln und/oder Subarachnoidalräumen über das zuführende Lumen und ein druckkontrolliertes kontinuierliches Drainieren von Exudaten über das abführende Lumen möglich, wobei die Messung des tatsächlichen intracraniellen Druckes im Flüssigkeitsbereich auch während der erfolgten Spülung zuverlässig ist.
Ein weiterer Vorteil wird darin gesehen, dass der erfindungsgemäße Katheter aus einem transparenten polymeren Werkstoff besteht, durch den jederzeit eine optische Kontrolle der zugeführten bzw abfließenden Fluide durch das zuständige medizinische Personal möglich ist. Weiterhin vorteilhaft wird gesehen, dass der erfindungsgemäße Katheter aus einem polymeren Werkstoff hergestellt ist, welcher röntgenkontrastfähig und/oder computertomographiefähig und/oder magnetresonanzfähig ist.

Es wurde überraschend gefunden, dass bei einer Dimensionierung des Durchmessers des zuführenden Lumens von maximal ein Drittel des Durchmessers des abführenden Lumens die Druckverhältnisse in den Hohlräumen zwischen den Hirnhäuten, in den Ventrikeln und/oder Subarachnoidalräumen bei der Zuführung und Abführung von Fluiden annähernd konstant sind.

Es wird weiterhin vorteilhaft gesehen, dass die Öffnungen der Lumen distal und/oder proximal zum Druckmesselement angeordnet sind, hierdurch wird die kontinuierliche Messung des tatsächlich wirkenden intracraniellen Druckes erreicht.

Durch die Anwendung des erfindungsgemäßen Katheters wird vorteilhafterweise erreicht, dass es für den Patienten zu keiner zusätzlichen Traumatisierung des Gehirns kommt, da bei der herkömmlichen Behandlung von Flüssigkeitsansammlungen in Hohlräumen zwischen den Hirnhäuten, in den Ventrikeln und/oder Subarachnoidalräumen, insbesondere bei intraventriculären Blutungen, Hydrocephalus, bei intracerebralen bzw. subduralen Hämatomen sowie bei Empyemen im Subduralraum immer zwei Trepanationslöcher in der Schädeldecke erforderlich sind, die durch die Verbindung mehrerer Funktionen im erfindungsgemäßen Katheter nicht mehr notwendig sind. Durch die Positionierung des Druckmesselementes im erfindungsgemäßen Katheter ist es jederzeit möglich, die Position des Katheters in den durch cerebrale Blutungen befallenen Hohlräumen zwischen den Hirnhäuten, Ventrikeln und/oder Subarachnoidalräumen sowie dem Hirngewebe zu bestimmen.

Der erfindungsgemäße Katheter hat eine hohe Anwendersicherheit, da durch diesen keine Konnektion auf herkömmliche Katheterverbindungselemente erforderlich ist und damit auch mögliche Leckagen auszuschließen sind.
Der erfindungsgemäße Katheter wird zur Behandlung von Flüssigkeitsansammlungen in Hohlräumen zwischen den Hirnhäuten, in Ventrikeln und/oder Subarachnoidalräumen, insbesondere bei intraventriculären Blutungen, Hydrocephalus unter permanenter Erfassung des dort herrschenden Druckes, des sog. intracraniellen Druckes, eingesetzt.
Dabei wird so verfahren, dass der Katheter in den zu behandelnden Hohlraum zwischen den Hirnhäuten, Ventrikel und/oder Subarachnoidalraum eingeführt und das freie Ende des Katheters im Blutungsbereich platziert wird.
Der vorliegende intracranielle Druck wird über das Druckmesselement erfasst und der am Druckaufnehmer ermittelte Wert definiert die maximal mögliche Zulaufmenge, die in den zu behandelnden Hohlraum zwischen den Hirnhäuten einführbar ist ohne Druckerhöhung.

Danach erfolgt die Zuführung der entsprechenden Fluide in Form von beispielsweise Kochsalzlösungen bzw. medikamentöse Lösungen über das zuführende Lumen und deren Öffnungen in den Hohlraum, in das Ventrikel und/oder Subarachnoidalräume unter permanenter Erfassung und Kontrolle des intracraniellen Druckes.

Nun wird entsprechend der Behandlungsmethode entweder eine Verdünnung herbeigeführt oder bei festen Blutclots lysiert, so dass das Abführen der nun entstehenden Fluide aus den Hohlräumen zwischen den Hirnhäuten, Ventrikeln und/oder Subarachnoidalräumen über die Öffnungen des abführenden Lumens in ein externes Speicherelement erfolgen kann.

Vorteilhaft wird hier gesehen, dass durch die Anwendung des erfindungsgemäßen Katheters zur nicht operativen Auflösung eines Blutclots durch Lysemedikamente, Verdünnung des Exudates und mechanisch aktive Ausspülung derselben erstmals möglich ist.
Dabei kann vorteilhaft die völlige Ausräumung des Blutungsbereiches in den Hohlräumen zwischen den Hirnhäuten, in Ventrikeln und/oder Subarachnoidalräumen erreicht werden, wobei dies sowohl mittels Schwerkraft als auch durch Pump- oder Saugelemente erfolgen kann, die jedoch immer unter Messung des herrschenden intracraniellen Druckes erfolgt. Der erfindungsgemäße Katheter und das Verfahren zu seiner Anwendung stellen ein autoregulatives Vorrichtungssystem dar, welches die Zuführung und Abführung von Fluiden in den Hohlräumen zwischen den Hirnhäuten, in Ventrikeln und/oder Subarachnoidalräumen mit dem Gesamtzustand des Patienten erfasst und reguliert. Ein weiterer Vorteil des erfindungsgemäßen Katheters und seiner Anwendung wird darin gesehen, dass durch die permanente Messung des intracraniellen Druckes in den zu behandelnden Hohlräumen zwischen den Hirnhäuten, Ventrikeln und/oder Subarachnoidalräumen die Sicherheit und die Gesundheit des Patienten jederzeit kontrollierbar und beeinflussbar ist.

Die Erfindung soll nun an einem diese nicht beschränkenden Ausführungsbeispiel näher beschrieben werden. Es zeigt:

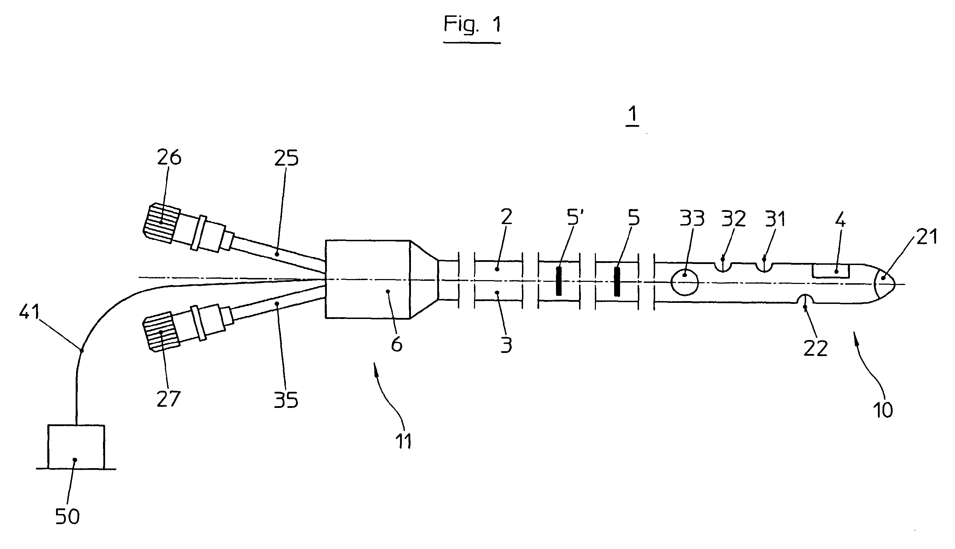

Figur 1: Katheter gemäß der Erfindung

In Figur 1 ist der erfindungsgemäße Katheter (1) dargestellt, der die Lumen (2,3) aufweist und aus einem polymeren transparenten Werkstoff hergestellt ist. Am freien Ende (10) des Katheters ist die Öffnung (21) des zuführenden Lumens (2) eingebracht. Von dieser Öffnung (21) beabstandet ist das Druckmesselement (4) positioniert, von dem wiederum beabstandet die Öffnungen (31, 32) des abführenden Lumens (3) eingebracht sind.

In diesem Ausführungsbeispiel sind weitere Öffnungen (22) des zuführenden Lumens (2) axial beabstandet zur Öffnung (21) eingebracht und eine zusätzliche Öffnung (33) des abführenden Lumens (3), welche radial zu den Öffnungen (31, 32) angeordnet ist.
Der Abstand der Öffnungen (21, 22, 31, 32, 33) zueinander entspricht in diesem Ausführungsbeispiel in etwa dem Doppelten ihrer äußeren Abmessungen.

Durch die Positionierung des Druckmesselementes (4) zwischen der Öffnung (21) des zuführenden Lumens (2) und den Öffnungen (31, 32) des abführenden Lumens (3) kann der intracranielle Druck beim Füllen und bei der Drainage Hohlräume zwischen den Hirnhäuten, der Ventrikel und/oder Subarachnoidalräume jederzeit genau gemessen und kontrolliert werden.

Das Druckmesselement (4) ist über elektrische bzw. optische Kabel (41) mit einem Druckmessgerät (50) verbindbar.

Die Lumen (2, 3) des Katheters (1) sind in diesem Ausführungsbeispiel einstückig miteinander verbunden und aus dem gleichen transparenten polymeren Werkstoff hergestellt.
Es liegt jedoch auch im Rahmen der Erfindung, dass der Katheter (1) Lumen (2, 3) aufweist, die aus unterschiedlichen Werkstoffen hergestellt sind. Dies kann beispielsweise ein härteres Material für das zuführende Lumen (2) sein, welches stabilisierend bei der Einführung des Katheters (1) in die zu behandelnden Hohlräume zwischen den Hirnhäuten, Ventrikel und/oder Subarachnoidalräume dient und ein weicheres Material für das abführende Lumen (3), welches sich der Geometrie in diesen anpasst.

Der erfindungsgemäße Katheter (1) weist in diesem Ausführungsbeispiel beginnend mit der Öffnung (33) Markierungselemente (5, 5) auf, die beispielsweise in einer metrischen Skalierung aufgebracht sein können und dem medizinischen Personal eine zusätzliche Information beim Einführen des Katheters in die zu behandelnden Ventrikel und/oder Subarachnoidalräume liefern.

Am freien Ende (11) des Katheters (1) ist ein Verbindungselement (6) dargestellt, in welchem die Lumen (2, 3) so fixiert werden, dass sie über die Schläuche (25, 35) zu den Anschlusselementen (26, 27) weiterleitbar sind, an die die entsprechenden Flüssigkeitszugabeelemente bzw. Flüssigkeitsspeicherelemente anschließbar sind. Das Verbindungselement (6) verhindert hier ein mögliches Trennen der Lumen (2, 3).

## Patentansprüche

1. Katheter für die Behandlung von Flüssigkeitsansammlungen in Hohlräumen zwischen den Hirnhäuten, in Ventrikeln und/oder Subarachnoidalräumen sowie dem Hirngewebe mit Fluiden, bestehend aus polymerem Werkstoff, wobei der Katheter (1) wenigstens zwei Lumen (2, 3) aufweist, wobei in jedes Lumen (2, 3) am freien Ende (10) des Katheters (1) wenigstens eine Öffnung (21, 31) eingebracht ist, wobei am freien Ende (10) des Katheters (1) ein Druckmesselement (4) positioniert ist, welches von den Öffnungen (21, 31) der Lumen (2, 3) beabstandet ist und wobei die Lumen (2, 3) so dimensioniert sind, dass die Druckverhältnisse in den Hohlräumen zwischen den Hirnhäuten, in den Ventrikeln und/oder Subarachnoidalräumen bei Zuführung und/oder Abführung von Fluiden annähernd konstant sind.

2. Katheter nach Anspruch 1, **dadurch gekennzeichnet, dass** der polymere Werkstoff transparent ist.

3. Katheter nach einem der vorhergehenden Ansprüche, **dadurch gekennzeichnet, dass** der polymere Werkstoff röntgenkontrastfähig und/oder computertomographiefähig und/oder magnetresonanzfähig ist.

4. Katheter nach einem der vorhergehenden Ansprüche, **dadurch gekennzeichnet, dass** der Durchmesser des zuführenden Lumens (2) maximal einem Drittel des Durchmessers des abführenden Lumens (3) entspricht.

5. Katheter nach einem der vorhergehenden Ansprüche, **dadurch gekennzeichnet, dass** die Öffnungen (21, 31) der Lumen (2, 3) distal und/oder proximal zum Druckmesselement (4) angeordnet sind.

6. Katheter nach einem der vorhergehenden Ansprüche, **dadurch gekennzeichnet, dass** weitere Öffnungen (22, 32, 33) axial in die Lumen (2, 3) eingebracht sind.

7. Katheter nach einem der vorhergehenden Ansprüche, **dadurch gekennzeichnet, dass** weitere Öffnungen (22, 32, 33) radial in die Lumen (2, 3) eingebracht sind.

8. Katheter nach einem der vorhergehenden Ansprüche, **dadurch gekennzeichnet, dass** die Öffnungen (21, 22, 31, 32, 33) der Lumen (2, 3) diametral gegenüberliegend angeordnet sind.

9. Katheter nach einem der vorhergehenden Ansprüche, **dadurch gekennzeichnet, dass** die Öffnungen (21, 22, 31, 32, 33) der Lumen (2, 3) alternierend gegenüberliegend angeordnet sind.

10. Katheter nach einem der vorhergehenden Ansprüche, **dadurch gekennzeichnet, dass** die Öffnungen (21, 22, 31, 32, 33) der Lumen (2, 3) einen Abstand zueinander aufweisen, der wenigstens ihren äußeren Abmessungen entspricht.

11. Katheter nach einem der vorhergehenden Ansprüche, **dadurch gekennzeichnet, dass** das Druckmesselement (4) ein Druckaufnehmer ist.

12. Katheter nach einem der vorhergehenden Ansprüche, **dadurch gekennzeichnet, dass** das Druckmesselement (4) über elektrische und/oder optische Kabel (41) mit einem Druckmessgerät (50) verbindbar ist.

13. Katheter nach einem der vorhergehenden Ansprüche, **dadurch gekennzeichnet, dass** der Katheter (1) beginnend am freien Ende (10) Markierungselemente (5,5') aufweist.

14. Verfahren zur Behandlung von Flüssigkeitsansammlungen in Hohlräumen zwischen den Hirnhäuten, in Ventrikeln und/oder Subarachnoidalräumen sowie dem Hirngewebe mit Fluiden, unter permanenter Erfassung des dort herrschenden Druckes, **gekennzeichnet durch** die folgenden Verfahrensschritte:
a) Einführen des Katheters in den Hohlraum
b) Erfassung des Umgebungsdruckes in dem Hohlraum
c) Zuführung von Fluiden über das zuführende Lumen (2) und die Öffnung (21) in dem Hohlraum unter Erfassung des Umgebungsdruckes
d) Abführen von Fluiden aus dem Hohlraum über die Öffnungen (31, 32) des abführenden Lumens (3) in ein externes Speicherelement unter Erfassung des dabei herrschenden Umgebungsdruckes.
